# EUROPÄISCHE PATENTANMELDUNG

(11) **EP 2 740 516 A2**
(43) Veröffentlichungstag der Anmeldung: **11.06.2014**
(21) Anmeldenummer: 13193236.0
(22) Anmeldetag: 18.11.2013
(51) Int. Cl.: A61N 1/375, H01R 24/58, H01R 13/52, H01R 13/187

(54) **Verfahren zur Herstellung einer mehrpoligen Steckerbohrung-Modulbaugruppe sowie danach hergestellte Steckerbohrung-Modulbaugruppe**

(30) Priorität: 04.12.2012 US 201261732949 P
(71) Anmelder: BIOTRONIK SE & Co. KG, 12359 Berlin (DE)
(72) Erfinder: Olbertz, Ralf, 12107 Berlin (DE); Lehmann, Stefan, 16269 Wriezen (DE); John von Freyend, Jörg, 13125 Berlin (DE); Litzke, Jan, 13507 Berlin (DE); von Grote, Markus, 12359 Berlin (DE)
(74) Vertreter: Lindner-Vogt, Karin L.

(57) **Zusammenfassung**

Ein Verfahren zur Herstellung einer mehrpoligen, insbesondere 4-poligen Steckerbohrung-Modulbaugruppe (1) eines Herzschrittmachers oder anderen implantierbaren kardiologischen Geräts, welche Modulbaugruppe (1) eine Steckeraufnahme (2), mehrere Kontaktfedernelemente (3), dazwischen angeordnete Dichtungen (4, 4') und einen Deckel (5) umfasst, weist folgende Verfahrensschritte auf:
- axiale Aneinanderreihung der Steckeraufnahme (2), der Kontaktfederelemente (3) mit dazwischen angeordneten Dichtungselementen (4, 4') und des Deckels (5) auf einem als Fertigungshilfsmittel dienenden Montagedom (7) zur Bildung einer vorbereiteten Baugruppe,
- Einlegen der vorbereiteten Baugruppe (1) in eine Spannvorrichtung (8),
- axiales Verspannen der vorbereiteten Baugruppe (1) mit einem definierten Verspannweg oder Spannkraft (F), und
- Einlegen der vorbereiteten, verspannten Baugruppe (1) in einen Vergussmaster (12) und deren Vergießen (13) mit einem Vergussmittel zur Bildung der Steckerbohrung-Modulbaugruppe (1).

## Beschreibung

Die Erfindung betrifft ein Verfahren zur Herstellung einer mehrpoligen, insbesondere vierpoligen Steckerbohrung-Modulbaugruppe eines Herzschrittmachers oder anderen implantierbaren kardiologischen Geräts, welche Modulbaugruppe eine Steckeraufnahme, mehrere Kontaktfedernelemente, dazwischen angeordnete Dichtungen und einen Deckel umfasst.

Zum Stand der Technik ist die US 7,601,033 B2 zu nennen, bei der eine solche Modulbaugruppe durch aufeinanderfolgendes Einschieben der entsprechenden Komponenten in Form von Dichtungen und Kontaktelementen in eine Montagehülse mit Hilfe eines jeweils wechselnden dornartigen Werkzeuges hergestellt wird.

Aus der US 7, 413,482 B2 ist es bekannt, eine solche Steckerbohrung-Modulbaugruppe durch Einsetzen von entsprechenden Kontakt- und Dichtungsringen in eine entsprechende Aufnahme vorzukonfektionieren und Kontaktfederzungen an die entsprechenden Kontaktringe zu deren elektrischer Kontaktierung anzubinden.

Die US 7,175,482 B2 offenbart einen zweistufigen Umspritzungsprozess von Kontaktierungselementen zur Fixierung insbesondere von Steckeraufnahmen, die Fixierschrauben für die jeweiligen Anschlusspins von Leitungen aufweisen.

In der US 7,717,754 B2 ist eine Steckerbohrung-Modulbaugruppe gezeigt, bei der wiederum Kontakt- und Dichtelemente sukzessive in eine entsprechenden Aufnahme eingesetzt und dort mit über entsprechende Füllkanäle eingebrachten Klebstoff fixiert werden. Das gleiche Bauprinzip offenbart die US 7,988,497 B2.

Aus der US 7,654,843 B2 ist es bekannt, die einzelnen Elemente einer Steckerbohrung-Modulbaugruppe in Form von Kontaktelementen, einer Steckeraufnahme und dazwischen angeordneten Dichtelementen auf einem Mandril in axialer Richtung aneinander zu reihen und diese Anordnung zu vergießen. Die Anschlusselemente für die Kontaktelemente können dabei vor oder nach dem Verguss an die Kontaktelemente angeschweißt werden.

Der Erfindung liegt die Aufgabe zugrunde, ein Herstellungsverfahren für eine mehrpolige, insbesondere vierpolige Steckerbohrung-Modulbaugpruppe sowie eine solche Steckerbohrung-Modulbaugruppe anzugeben, bei der mit Hilfe eines definierten Produktionsprozesses und einer entsprechenden Ausbildung der Dichtungselemente eine besonders zuverlässige, gegen feuchte Medien auch bei Hochspannungsanwendungen resistente und dichte Konstruktion zur Anwendung in implantierbaren kardiologischen Geräten geschaffen wird.

Diese Aufgabe wird durch das Verfahren des Anspruches 1 bzw. durch die im Anspruch 9 angegebene, damit hergestellte Steckerbohrung-Modulbaugruppe gelöst. Die im Kennzeichnungsteil des Anspruches 1 angegebenen Herstellungsschritte umfassen:
- axiale Aneinanderreihung einer Steckeraufnahme, von Kontaktfederelementen mit dazwischen angeordneten Dichtungselementen und eines Deckels auf einem als Fertigungshilfsmittel dienenden Montagedom vorzugsweise mit einem Zwischenring zur Bildung einer vorbereiteten Baugruppe,
- Einlegen der vorbereiteten Baugruppe in eine Spannvorrichtung,
- axiales Verspannen der vorbereiteten Baugruppe mit einem definierten Verspannweg oder Spannkraft,
- Einlegen der vorbereiteten, verspannten Baugruppe in einen Vergussmaster, und
- deren Vergießen mit einem Harz zur Bildung der Steckerbohrung-Modulbaugruppe.

Die Montage der Steckerbohrung-Modulbaugruppe wird verwendet, um ein neues Kontaktierungssystem sowie neu entwickelte Montagefunktionen/-prozesse der Einzelkomponenten zur Anwendung bei Schrittmachern (SM) bzw. anderen implantierbaren medizinischen Geräten (ICD) zu implementieren. Ähnlich wie im bekannten IS-1/DF-1 System können die elektrisch aktiven Implantate mit Elektroden kontaktiert werden.

Die Steckerbohrung-Modulbaugruppe bildet auf einem SM bzw. ICD die Steckerbohrung ab. Mit dieser kann der entsprechende Stecker einer Elektrode aufgenommen werden. Die Positionen der Einzelkomponenten, die die Steckerbohrung-Modulbaugruppe bilden, sind durch die Norm ISO 27168 genormt.

Das Montagekonzept sieht vor, die Einzelkomponenten aneinander zu reihen und über einen Kraft-Wege-Montageprozess diese zu verspannen. Über diesen Montageprozess wird zum einem insbesondere erreicht, dass die Dichtungen axial verpresst werden. Des Weiteren ist dieser Montageprozess geeignet, um die elektrische Potentialtrennung gegen Spannungen sicher zustellen, um gegen Feuchtemedien zu isolieren und um die Einzelkomponenten auf ihre Positionen zu bringen.

Zur Erstellung der Steckerbohrung-Modulbaugruppe wird jede einzelne Komponente mit einem speziellen Werkzeug aufgenommen und auf das als Dom ausgebildete Fertigungshilfsmittel (im Folgenden "FHM") aneinandergereiht. Die vorbereitete Baugruppe wird vorzugsweise in eine Vorrichtung eingelegt, um den definierten Verspannweg im Bereich von 0,01 mm - 5 mm und/oder einer Spannkraft von bis zu 200 N zu verfahren. Bei Erreichen der maximalen Kraft regelt ggf. der Verfahrweg ab, um die Komponenten nicht zu beschädigen. Somit wird die Position jeder Einzelkomponente sichergestellt und die Funktion der axialen Dichtungsfunktion wird umgesetzt.

Anschließend wird die vorbereitete Baugruppe in einen Vergussmaster eingelegt, der mit dem Harz vorzugsweise mit oder ohne Vakuum, im Bereich zwischen 1100 mbar und 0,1 mbar befüllt wird.

In weiteren Ausführungsformen wird der Vergussmaster mit Polyurethan, Silikon oder deren Gemischen gefüllt.

Unabhängig, ob es sich bei der Steckerbohrung-Modulbaugruppe um eine DF-4 oder IS-4 Baugruppe handelt, lässt es sich variabel auf einem SM oder anderweitigen ICD montieren. Eine durch das erfindungsgemäße Konzept mögliche funktionelle Prüfung (Dichtigkeit/HV-Messung/Steck-/Auszugskräfte) und visuelle Prüfung vor der Montage bedeutet zudem, dass bei einem auftretenden Fehlermerkmal geringere Ausschlusskosten anfallen, da der Träger (SM/ICD) ohne Schädigung erhalten bleibt. Erst nach einer fehlerfreien Testung kommt es zu einer Montage der Baugruppe auf ein elektrisch aktives Implantat.

Bevorzugte Weiterbildungen im Rahmen des erfindungsgemäßen Verfahrens betreffen die Dichtungselemente zwischen den Kontaktfederelementen innerhalb der Steckerbohrung, welche Dichtungen gegen das Eindringen von feuchten Medien und zur Potentialtrennung von elektrischen Spannungen im Hochspannungs- bzw. Niederspannungsbereich zwischen mindestens zwei oder mehreren elektrischen Kontakten fungieren. Sie erfüllen auch weitere Anforderungen, wie z.B. die Umsetzung von geringen Einsteck-/Auszugskräften.

Die Außendichtung kann aus einer oder mehreren Dichtsegmenten/ Flächen bestehen. Im Rahmen dieser Außendichtung können die Dichtungselemente vorzugsweise zur Realisierung einer radialen Vorspannung in eine zylindrische Bohrung einer Hülse oder eines Moduls einmontiert werden.

Bevor auf die bevorzugten Weiterbildungen gemäß den Ansprüchen 6 und 7 bezüglich eines Gewindestiftes in der Steckeraufnahme der Modulbaugruppe eingegangen wird, sollen die weiteren verfahrens- und vorrichtungstechnischen Aspekte im Zusammenhang mit den Weiterbildungen der Dichtungen abgehandelt werden.

So kann ein Dichtelement zwar ohne Spacer realisiert werden, das durch einen einfachen Spritzgussprozess herzustellen ist.

Bevorzugt kommt in dem Dichtelement ein Spacer zu Anwendung. Es kann dabei entweder auf einen einfachen Spritzgussprozess mit Einlegeteil (Spacer) zurückgegriffen werden oder es wird auf ein 2K-Spritzgußprozess zurückgegriffen, um die auf dem Spacer angeordnete Dichtung in Form eines Elastomerelements zu realisieren. Findet ein Einlegeteil (Spacer) Verwendung, so kann zur Erhöhung des Haftverhaltens zwischen Einlegeteil (Spacer) und Dichtung, das Einlegeteil vorbehandelt werden.

Bei Bedarf kann zur Verringerung der Steckkräfte eine Beschichtung zumindest im Bereich der Innendichtung angebracht werden, die mittels Tauch- bzw. Sprühprozess aufgetragen wird.

Das Dichtungselement mit bis zu drei voneinander unabhängigen Dichtungsfunktionen verhindert das Eindringen von Flüssigkeiten und dient zur Potentialtrennung von niedrigen/hohen Spannungen im Bereich von 0 bis 2500 Volt zwischen mindestens zwei oder mehr elektrischen Kontakten und schützt gegen weitere elektrische Potentiale wie durch Feuchtemedien gebildete.

Sekundär unterstützt der integrierte Spacer die Unabhängigkeit der drei Dichtungsfunktionen und dient zudem als Abstandshalter.

Beschrieben ist eine Baugruppe aus zwei Komponenten, dem Spacer (PEEK) und einem angespritztem Elastomer (Silikon) als eigentliche Dichtung in individueller Shorehärte. Diese Baugruppe wird als Dichtungselement bezeichnet. Die Dichtung beinhaltet drei voneinander unabhängige Dichtfunktionen, die aus der Außendichtung, der Innendichtung und der axialen Dichtung bestehen. Alle drei Dichtfunktionen erfüllen primär die gleichen Anforderungen, nämlich Dichtigkeit gegen feuchte Medien und elektrische Potentialtrennung gegen Spannungen im Hochspannungsbereich (DF-4/> 800 V) bzw. Niederspannungsbereich (IS-4/< 800 V). Sekundär werden weitere individuelle Anforderungen, wie Einhaltung der Einsteck-/Auszugskräfte, an die einzelne Dichtfunktion gestellt und erfüllt.

Bei einem Prozess, der das Elastomer und Plastomer (Werkstoffe s. u.) miteinander haftend vernetzt, ist auch die Realisierung von Technologien, wie Spritzprozessen bzw. Vergussprozessen zu berücksichtigen. Ein alternativer Werkstoff für eine der beiden Komponenten oder auch völlig alternative Materialpaarungen sind möglich.

Eine Variante der Dichtung kann auch auf die Funktion der Außendichtung verzichten, sofern zwischen Dichtung und Spacer eine chemisch haftende Verbindung besteht und eine solche zur weiteren Umgebung erzeugt werden kann. Parallel können die Positionsabstände der einzelnen Elemente in der Baugruppe nach Norm eindeutiger eingestellt werden, da der Spacer über einen festen Anschlag verfügen kann.

Wenn die technischen Anforderungen an das System erfüllt werden, so ist auch eine Komponente Dichtung ohne Spacer möglich und diese besteht folglich nur aus einem oder mehreren Elastomeren (Silikonhärte/n).

Die Funktionsanforderungen an die Außendichtung sind in zwei Untergruppen zu gliedern, bei denen es sich um den Einsatz im Hochspannungs- bzw. Niederspannungsbereich handelt.

Im Niederspannungsbereich kann auf eine Vorspannung zurückgegriffen werden, welche ein Eindringen von feuchten Medien, sowie Spannungsüberschläge verhindert. Da jedoch nur geringe Stromspannungen auftreten, kann auch auf einen direkten Umspritz-/Vergussprozess von Elastomeren/Plastomeren zurückgegriffen werden.

Im Hochspannungsbereich (> 800V) muss eine Vorspannung umgesetzt werden, die durch einen Montageprozess in eine zylindrische Bohrung realisiert wird. Dabei ist es unabhängig, ob es sich dabei um eine Bohrung eines Moduls oder einen einzelnen Ring handelt, der übermontiert wird. Die Außendichtung sollte weiter einen langen Kriechweg gegen ein Eindringen von feuchten Medien und eine geeignete Montagegeometrie aufweisen.

Nur mit Umsetzung dieser Anforderung kann sichergestellt werden, dass es in der Funktion Außendichtung zu keinem Spannungsüberschlag zwischen den elektrischen Kontakten kommt.

Sofern die Anforderungen der Vorspannung umgesetzt werden, beispielsweise durch einen Prozess, der das Elastomer und Plastomer miteinander haftend vernetzt, ist auch die Realisierung von Technologien wie Spritzprozessen bzw. Vergussprozessen zu berücksichtigen. Ein alternativer Werkstoff für eine der beiden Komponenten oder auch andere alternative Materialpaarungen sind möglich.

Hülsen werden verwendet, um die Vorspannung der äußeren Dichtlippen der Dichtung zu realisieren. Der Hülsen-Innendurchmesser ist dabei kleiner als der größte Außendurchmesser der Dichtung.

Die Hülse wird durch einen Spritz-, Verguss- oder Tiefziehprozess, als Dreh- oder Pressteil hergestellt, dessen Innendurchmesser geringer als der Außendurchmesser der Dichtung ist. Die Werkstoffe können aus Epoxydharz, PEEK, Polysulfon, Polyurethan, PolyurethanCopolymer, Silikon, Keramik, Polyimid und/oder deren Mischformen bestehen. Die Hülse wird nach einer Reinigung und optischen Prüfung mit Hilfe eines Hilfswerkzeuges händisch oder automatisiert auf die Dichtung geschoben. Die Außendichtung kann aus einer oder mehreren Dichtsegmenten/Flächen bestehen. Die Dichtung kann mit und ohne Vorspannung im Außendichtlippenbereich belastet sein. Bei einer Verspannung zwischen den Außendichtlippen und der Hülse liegt die optimale Vorspannung zwischen 0,01mm und 1 mm.

Mit der Umsetzung der Vorspannung auf die äußere Dichtungsfunktion, welche nur durch eine Hülse bzw. einen anderen zylindrischen Teilabschnitt realisiert ist, wird auch die Sicherheit gegenüber Spannungsüberschlägen, Undichtigkeit etc. gewährleistet.

Die Funktionsanforderungen an die Innendichtung sind für den Einsatz im Hochspannungs- bzw. Niederspannungsbereich identisch. Es muss eine Potentialtrennung zwischen den elektrischen Kontakten und auch zwischen Dichtung und feuchten Medien gewährleistet werden. Dabei handelt es sich um zwei Einzeldichtungen, in einem definierten Abstand, der jedoch variabel gewählt werden kann. Die Anzahl der Einzeldichtungen kann auf eine Einzeldichtung verringert bzw. auf mehr als zwei Einzeldichtungen erhöht werden.

Die Einzeldichtung können je nach Anwendung/Anforderung im Querschnitt identisch bzw. unterschiedlich ausgeführt werden. Der Querschnitt der Einzeldichtung kann parallel oder auch konisch sein. Der Anstellwinkel ist variabel.

Der Innendurchmesser der Innendichtung, der Durchmesser zwischen den Einzeldichtungen, sowie die konischen angrenzenden Innenflächen sind auf Funktionen/Anforderungen ausgelegt und sind hinsichtlich der Abmaße als Variable anzusehen.

Eine weitere Anforderung an die Funktion Innendichtung besteht darin, geringe Einsteck- bzw. Auszugskräfte für die später in die Steckerbohrung einzusetzenden und daraus herauszuziehenden Stecker umzusetzen, ohne dabei die erstgenannten Anforderungen zu verletzen. Bei Bedarf kann zur Verringerung der Steckkräfte eine Beschichtung beispielsweise auf Basis eines Silikon-Gleitmittels aufgebracht werden, die mittels einem Tauch- bzw. Sprühprozess aufgetragen wird.

Die Funktionsanforderungen der Axialdichtung sind für den Einsatz im Hochspannungs- bzw. Niederspannungsbereich identisch. Dabei wird ebenfalls wieder gegen das Eindringen von feuchten Medien und zur Potentialtrennung zwischen den elektrischen Kontakten abgedichtet.

Diese Axialdichtung besteht an der Komponente Dichtung beidseitig und kann vorzugsweise konvex ausgelegt werden. Eine andere Geometrie bzw. eine variable konvexe Höhe ist ebenfalls möglich. Die Funktion der Dichtung wird erst durch die Montage mit weiteren Modulkomponenten der Gesamtbaugruppe Modul erzielt, wenn diese axial verspannt werden. Die Dichtung dichtet dabei direkt gegen ein Kontaktelement oder gegen ein Plastomer ab.

Der Spacer verfügt über Anzahl von Durchbrüchen, durch die das Elastomer gespritzt wird. Dieses konstruktive Konzept unterstützt die mechanische Verbindung zwischen Elastomer und Plastomer, da es bei den derzeitig verwendeten Werkstoffen zu keiner eindeutigen Vernetzung/Haftung kommen kann. Der Innen- und Außendurchmesser, sowie die axiale Länge des Spacers, sind individuell auf das Elastomer und den voneinander unabhängigen Dichtungsfunktionen variabel abgestimmt.

Zur Verbesserung des Haftvermögens zwischen Elastomer (Dichtung) und Spacer können vorbereitende Prozesse wie z.B. Definition der Oberflächenrauheit, Plasmaaktivieren, nasschemisches Benetzen (mit Primem oder ähnlichem), CO₂-Strahlen, etc. zur Anwendung kommen. Alternativ kann der Spacer auch aus einem Elastomer in einer deutlich anderen Shorehärte, bestehen.

Folgende Werkstoffe/Prozessparameter sind bevorzugt anzugeben:
Für die Dichtung aus Silikon wird vorzugsweise ein Zwei-Komponenten-Gemisch verwendet, welches biokompatibel sein muss. Die Shorehärten, die zum Einsatz kommen können, belaufen sich auf 20 - 70 Shore A. Es wird eine Tempertemperatur von 150 - 230°C und eine Temperzeit von 2 - 10 h benötigt. Über das Zusammenspiel Tempertemperatur/-zeit wird die Härte des Silikons eingestellt.

Der Spacer kann aus einem Epoxydharz, PEEK, Polysulfon, Polyurethan, Polyurethan Copolymer, Silikon, Keramik, Polyimide und/oder deren Mischformen bestehen.

Durch geeignete vorbereitende Prozesse oder unter Verwendung anderer geeigneter Werkstoffe kann eine bessere Haftung erzielt werden.

Durch Verwendung von Nano-Kristallen als eine Art Heftklammern können an sich schlecht aneinander haftende Materialien, wie hier Silikon und Teflon aufeinander haften. Verzichtet wird auf chemische Klebstoffe. Diese Technik ist einfach anzuwenden und bedarf keiner teuren Spezialausstattung oder -materialien.

Die Heftklammern sind Kristalle aus beispielsweise Zinkoxid mit einer Größe von mehreren Nano- bis wenigen Mikrometern. Sie können die Form von Tetrapoden - vier starren Armen, die von einem zentralen Punkt ausgehen - aufweisen. Beim Aneinanderfügen von Polymeren, wie Teflon und Silikon, werden zunächst Zinkoxidkristalle gleichmäßig auf einer erwärmten Teflonschicht verteilt. Darüber wird eine Silikonschicht aufgebracht. Um die beiden Materialien zu verbinden, werden sie für weniger als eine Stunde auf etwa einhundert Grad Celsius erwärmt.

Dieses Verfahren ist gesundheitlich absolut unbedenklich, also biokompatibel. Werden Materialien aneinander geklebt, können demgegenüber chemische Reaktionen und Prozesse die Polymere verändern und sie im ungünstigsten Falle sogar giftig machen. Die "Tetrapoden-Heftklammern" hingegen stellen eine rein mechanische Verknüpfungstechnik dar.

Die Steckerbohrung-Modulbaugruppe beinhaltet im Bereich der Steckeraufnahme auch einen Gewindestift mit durchgehender Bohrung, welche der Entlüftung während des Montageprozess und dem Druckabbau beim Stecken eines Elektrodensteckers dient.

Durch die durchgehende Entlüftungsbohrung im Gewindestift entsteht am Gewindeauslauf eine Ringschneide, die beim Fixieren des Elektrodensteckers bzw. des Montagedoms, durch das Aufbringen hoher Drehmomente in der Größenordnung von 1 bis 50 Ncm eine Deformation erfährt. Dies bewirkt höhere Fixierungskräfte des Kontaktierungssystems und bietet zudem ein Indiz über eine Kontaktierung bzw. Fehlkontaktierung.

Der Gewindestift wird im Montageprozess für die Steckerbohrung-Modulbaugruppe eingesetzt und weist eine innenliegende Entlüftungsbohrung auf. Bei fertig gestellter Steckerbohrung-Modulbaugruppe auf einem Produkt (SM/ICD/Patientenkabel/Adapter) kommt ein Gewindestift mit gleicher Funktionalität zum Einsatz.

Im Montageprozess kommt als Fertighilfsmittel (FHM) ein Dom zum Einsatz. Auf diesem werden alle Einzelkomponenten, die zur Umsetzung der Steckerbohrung-Modulbaugruppe benötigt werden, axial aufgesetzt und über ein definiertes Kraft-Wegverhältnis verpresst. Bei Wechsel von FHM (Dom) oder beim Stecken von Prüfkörpern und Elektrodenstecken wird in der Steckerbohrung ein Druck aufgebaut, der so groß sein kann, dass das gesteckte Element wieder entgegen der Einsteckrichtung herausgedrückt wird. Die Durchgangsbohrung ermöglicht es dem angestauten Druck entweichen zu können. Die gesteckten Elemente neigen so nicht mehr dazu, unkontrolliert aus der Steckerbohrung-Modulbaugruppe zu rutschen, also herausgedrückt zu werden.

Der Gewindestift wird spanend oder durch Umformen hergestellt. Drück-/Räumwerkzeuge können die Innenbohrung bzw. den Innensechskant umsetzen. Ätzverfahren werden bei Bedarf zum Entgraten und/oder zur Verbesserung der Oberfläche eingesetzt, beispielsweise um eine verbesserte Haftung zum angrenzenden Werkstoff zu erzielen.

Das Federelement verfügt vorzugsweise über ein Schweiß-/Lötplateau, um mit einem Schweiß-/Lötparametersatz in einer Ebene zur Steckeraufnahme Bänder anschweißen zu können. Je nach Wahl eines Verbindungsprozesses kann dies mit oder ohne Platine ausgeführt werden. Die Platine wird direkt auf der Komponente Federelement angebracht. Diese Trägerfläche (Platine) kann dazu dienen, die Signalleitungen aufzunehmen oder nur rein als Fixierung bzw. Abschirmung gegenüber anderen Bauteilen.

Das Schweißplateau der Komponente Federelement kann sich auf einem Höhenniveau zur Schweißfläche der Steckeraufnahme befinden.

Das Schweißplateau der Komponente Federelement kann direkt bei dem Herstellungsprozess dessen Federkorpus berücksichtigt werden. Dieses ist dann durch spanende Herstellungsprozesse wie Drehen und Fräsen zu berücksichtigen. Alternative Prozesse wären auch Umformen und Sintern. Das Schweiß-/Lötplateau kann auch als separates Element gefertigt werden und an die Komponente Federelement angefügt werden.

Besteht Bedarf an lötfähigen Platinen, so werden diese nach üblichen Herstellungsprozessen gefertigt und mit der Komponente Federelement verbunden.

Ein paralleles Schweißen/Löten aller Verbindungen ist möglich. Dies ermöglicht eine wesentlich kürzere Taktzeit, die auch automatisiert ausgeführt werden kann, was aus wirtschaftlicher Sicht vorteilhaft sein kann.

Das Schweißplateau auf dem Federelement dient der Aufnahme einer externen Kontaktierungsebene, die die Signale von der Elektrode über das Federelement zur Verarbeitungsebene in den Schrittmacher transportiert. Es können sich auf dem Federelement ein oder mehrere Plateaus befinden bzw. kann das Federelement ganz vierkantig sein. Die Vorteile einer flachen gegenüber einer runden Fläche liegen in der besseren und sicheren Verbindungsmöglichkeit beim Löten, Lasern, Kleben, Crimpen. Die Werkstoffe sind MP-35N, 316L, Titan, Tantal, Wolfram, Niob. Diese können auch beschichtet sein, um für die oben genannten Fügeverfahren die notwendige Grundlage zu bieten. Auch besteht ein wichtiger Vorteil eines flachen Plateaus gegenüber einer reinen runden Federhülse darin, das diese im bereits montierten Zustand auf eine Trägerfläche im Fügeverfahren montiert werden kann.

Die Größe des Plateaus kann in der Breite und der Länge in Abhängigkeit von der Größe des Federkäfigs schwanken. Das Plateau kann auch nachträglich auf eine runde Hülse durch ein Fügeverfahren aufgebracht werden.

Bei einer alternativen Anbindung in Form einer Platine kann die Trägerfläche dazu dienen die Signalleitungen aufzunehmen oder nur rein als Fixierung bzw. Abschirmung gegenüber anderen Bauteilen wirken. Trägerflächen mit Signalleitung können verschiedene Arten von Schaltkreissubstraten, Elastomeren, Thermoplasten, Plastomeren und/oder Keramik sein. Trägerflächen ohne Signalleitung können Epoxydharze, PEEK, PU, Polysulfon, Keramik etc. sein.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1: einen Axialschnitt durch eine Steckerbohrung-Modulbaugruppe in einem Fertigungszwischenschritt,
- Fig. 2: einen Axialschnitt analog Fig. 1 nach dem Verguss der Modulbaugruppe,
- Fig. 3: eine Explosionsdarstellung der Einzelkomponenten der Modulbaugruppe,
- Fig. 4: eine perspektivische Ansicht einer Dichtung in einer ersten Ausführungsform,
- Fig. 5: eine perspektivische Ansicht der Dichtung gemäß Fig. 4 nach Art einer Explosionsdarstellung,
- Fig. 6 und 7: perspektivische Ansichten einer Dichtung analog Fig. 4 und 5 in einer zweiten Ausführungsform,
- Fig. 8 und 9: perspektivische, teilweise weggebrochene Ansicht sowie einen Axialschnitt der Dichtung gemäß den Fig. 6 und 7,
- Fig. 10: eine perspektivische Ansicht der Dichtung gemäß Fig. 4 mit hervorgehobenen Außendicht-Flächen,
- Fig. 11 und 12: perspektivische Ansichten einer in einer Hülse vorgespannten Dichtung gemäß Fig. 10 in Gesamt- und teilweise weggebrochener Darstellung,
- Fig. 13: eine perspektivische Ansicht der Dichtung gemäß Fig. 4 mit hervorgehobenen Innendicht-Flächen,
- Fig. 14 und 15: Querschnitte der Dichtung gemäß Fig. 13 in zwei unterschiedlichen Ausführungsformen der Innendicht-Lippen,
- Fig. 16: eine perspektivische Darstellung des Dichtelementes gemäß Fig. 4 mit hervorgehobenen Axial-Dichtflächen,
- Fig. 17 und 18: eine perspektivische, teilweise weggebrochene Darstellung eines Gewindestiftes in zwei unterschiedlichen Ausführungsformen,
- Fig. 19: eine Darstellung der Kontaktelemente der Modulbaugruppe mit angeschweißten Kontaktbändern, und
- Fig. 20: eine perspektivische Ansicht der Kontaktelemente der Modulbaugruppe in Zuordnung zu einer Platine.

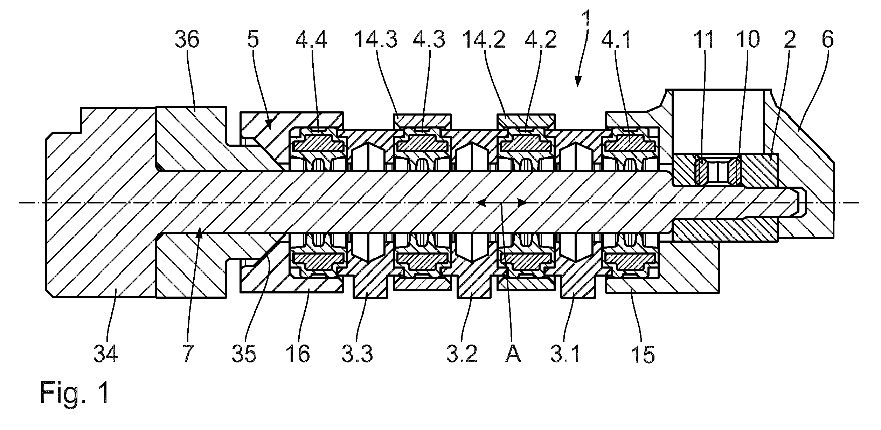

Wie aus Fig. 1 deutlich wird, wird eine als Ganzes mit 1 bezeichnete Steckerbohrung-Modulbaugruppe in vierpoliger Ausführung - eine sog. "four-pole-connector-cavity"-gebildet aus einer Steckeraufnahme 2, sich in axialer Richtung A abwechselnd aneinanderreihende Kontaktfederelemente 3.1 bis 3.3. und Dichtungselemente 4.1 bis 4.4 sowie einen Deckel 5. Die Steckeraufnahme 2 ist in einem Sockelteil 6 angeordnet. Dabei sind die inneren Dichtelemente 4.2, 4.3 jeweils in Ringhülsen 14.2, 14.3 eingepresst, wodurch die im Folgenden noch näher erläuterte Außendichtung erfolgt. Die äußeren Dichtelemente 4.1, 4.4 sitzen in entsprechenden ringförmig umlaufenden Schulterabschnitten 15, 16 am Sockelteil 6 bzw. am Deckel 5. Auch damit ist ein radiales Verpressen der Dichtelemente 4.1, 4.4 und eine gute Abdichtung in diesem Außenbereich zu realisieren.

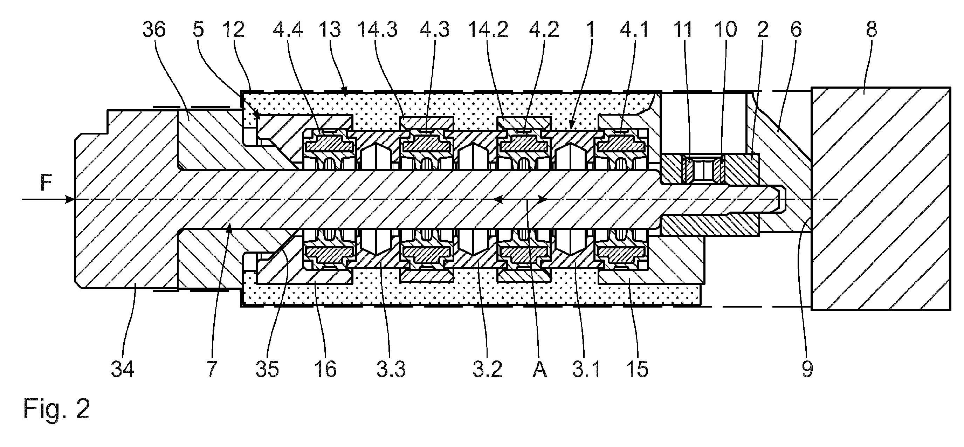

Die Komponenten Steckeraufnahme 2, Kontaktfederelemente 3.1 bis 3.3, Dichtelemente 4.1 bis 4.4 und Deckel 5 werden gemeinsam auf einen als Fertigungshilfsmittel dienenden Montagedom 7 aufgereiht, so dass eine vorbereitete Baugruppe gebildet ist. Der Montagedom 7 weist einen stufenförmig abgesetzten Kopf 34 auf, der einen mit dem Deckel 5 über Schrägschultern 35 in Eingriff stehenden Zwischenring 36 aus PTFE als weiteres Fertigungshilfsmittel beaufschlagt. Der Montagedom 7 mit Zwischenring 36 wird mit der vorbereiteten Baugruppe aus Steckeraufnahme 2, Kontaktfederelemente 3.1 bis 3.3, Dichtelemente 4.1 bis 4.4 und Deckel 5 in eine in Fig. 2 schematisch dargestellte Spannvorrichtung 8 eingelegt, wobei der Montagedom 7 mit einer maximalen Spannkraft F von 200 N verspannt wird. Die Spannvorrichtung 8 weist einen Anschlag 9 auf, gegen den die vorbereitete Baugruppe 1 mit Hilfe des Montagedoms 7 verspannbar ist. Mit Hilfe des in der Gewindebohrung 10 der Steckeraufnahme 2 eingeschraubten Gewindestiftes 11 wird in der verspannten Position der Montagedom 7 relativ zur Steckeraufnahme 2 fixiert, so dass die Verspannung der Elemente der Baugruppe 1 gesichert wird.

Statt der definierten Verspannung mit der Spannkraft F kann das axiale Verspannen auch mit einem definierten Verspannweg beispielsweise im Bereich zwischen 0,01 mm und maximal 5 mm erfolgen. In jedem Falle ist das axiale Verspannen der vorbereiteten Baugruppe Maximalwert-begrenzt.

Die so verspannte Baugruppe 1, wie sie in Fig. 1 dargestellt ist, wird anschließend in einen in Fig. 2 gestrichelt dargestellten Vergussmaster eingelegt und mit einem Verguss 13 (punktiert angedeutet in Fig. 2) aus einem geeigneten Harz umhüllt. Damit ist die Steckerbohrung-Modulbaugruppe 1 zur weiteren Verarbeitung fertig konfektioniert, wie sie am Ende der folgenden Beschreibung der verschiedenen Ausführungsbeispiele der einzelnen Elemente der Modulbaugruppe noch näher erläutert ist. Der Verguss der Modulbaugruppe 1 innerhalb des Vergussmasters 12 kann mit oder ohne Vakuumeinfluss in einem Druckbereich zwischen 1100 mbar und 0,1 mbar erfolgen.

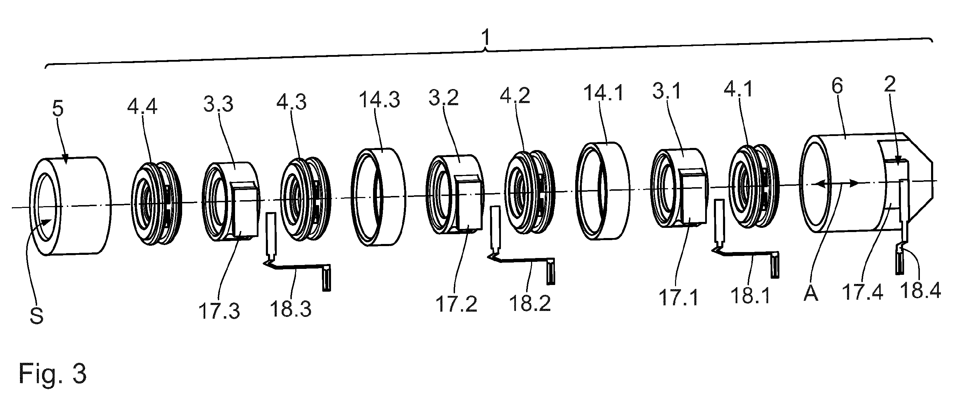

In Fig. 3 ist die Aneinanderreihung der einzelnen Elemente der Modulbaugruppe 1 aufgrund der Explosionsdarstellung gut erkennbar. Wie ebenfalls aus Fig. 3 hervorgeht, weisen die Kontaktfederelemente 3.1 bis 3.3 von ihrem Außenumfang abstehende Plateaus 17.1 bis 17.3 auf, die als ebene Lötflächen für die schematisch dargestellten Kontaktbänder 18.1 bis 18.3 zum elektrischen Anschluss der Kontaktfederelemente 3.1 bis 3.3 ausgebildet sind. Beim Verguss 13 der Modulbaugruppe 1 bleiben diese Plateaus 17.1 bis 17.3 und das Plateau 17.4 der Steckeraufnahme 2 vom Harz frei, so dass an ihnen problemlos die Kontaktbänder 18.1 bis 18.4 für den weiteren Verbau beispielsweise im Kopfanschlussstück (Header) eines implantierbaren kardiologischen Gerätes angebracht werden können.

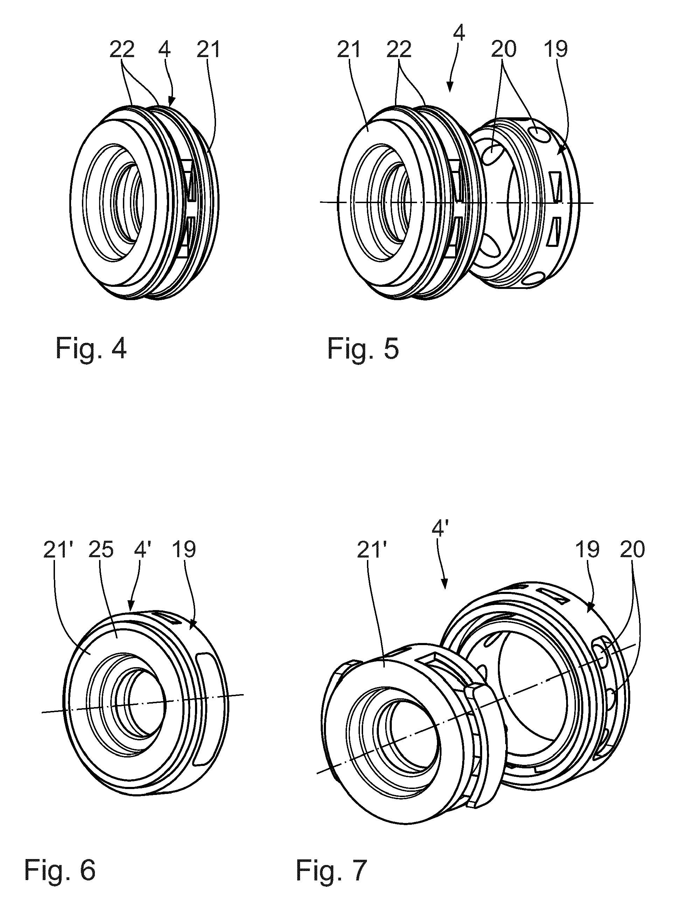

In den Fig. 4 und 5 ist eine erste Ausführungsform der Dichtelemente 4.1 bis 4.4 dargestellt. Bei diesem Dichtelement, das im Folgenden pauschal mit Ziffer "4" bezeichnet werden soll, ist als harter Kern ein Spacer 19 vorgesehen, der Durchbrüche 20 für das umspritzte Material der eigentlichen Dichtung 21 aufweist. Der Spacer 19 ist aus einem relativ harten Plastomer gefertigt. Die ihn umgebende Dichtung 21 besteht aus einem Elastomer, beispielsweise aus einem Silikon-Werkstoff mit einer Shore-Härte von 20 bis 70 Shore A. Der Spacer 19 selbst kann noch zur Verbesserung des Haftvermögens an seiner Oberfläche durch bekannte Prozesse, wie eine bestimmte definierte Oberflächenrauheit, durch Plasmaaktivieren, nasschemisches Benetzen oder Kohlendioxid-Strahlen so aufbereitet sein, dass die Haftung zwischen ihm und dem Material der Dichtung 21 verbessert wird.

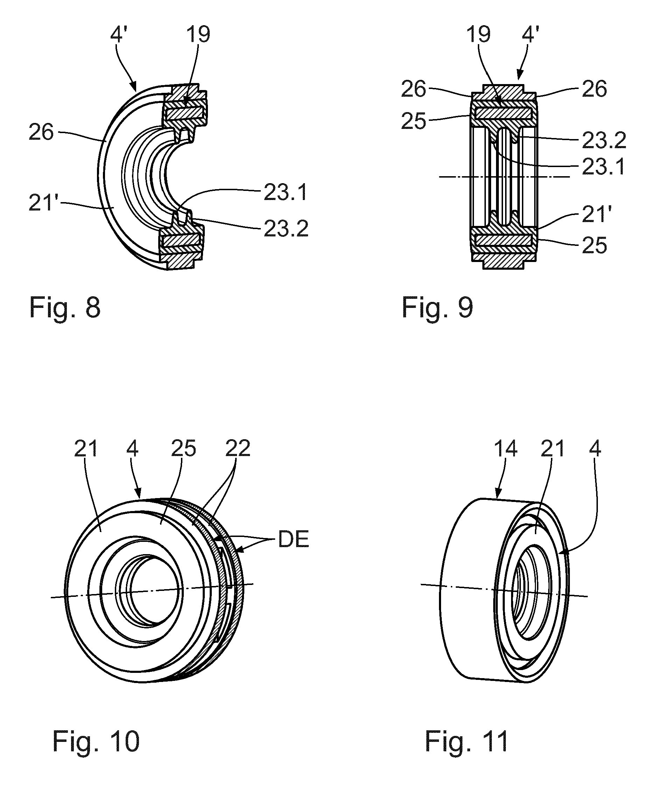

In Fig. 6 bis 9 ist eine alternative Ausführungsform für das Dichtelement dargestellt, die mit 4' bezeichnet wird. Darin ist ein wiederum mit Durchbrüchen 20 versehener Spacer 19 so mit einer angespritzten Dichtung 21' versehen, dass am Außenumfang des Dichtelementes 4' kein Material der Dichtung 21' vorhanden ist. Die sich daraus ergebenden Folgerungen für das Dichtungsverhalten werden im Folgenden anhand der Fig. 10 bis 18 näher erläutert.

In Fig. 10 bis 12 ist die Funktion der in Fig. 10 schraffiert angedeuteten Außendichtung DE des Dichtelementes 4 gezeigt. Dazu weist die elastomere Dichtung 21 auf ihrer Außenseite zwei umlaufende Ringschultern 22 als Funktionsfläche zur Abdichtung auf. Das Dichtelement 4 ist in eine Ringhülse 14 eingeschoben, so dass die Dichtung 21 radial nach innen beaufschlagt ist und mit den Ringschultern 22 damit die Außendichtung DE bildet und dicht gegen die Innenseite der Ringhülse 14 abdichtet. Damit ist das Dichtelement 4 besonders gut für den Hochspannungsbereich größer 800 Volt geeignet, wie in der Beschreibungseinleitung umfassend dargelegt wurde. Damit wird entsprechenden Spannungsüberschlägen zwischen den elektrischen Kontaktfederelementen 3.1 bis 3.3 vorgebeugt.

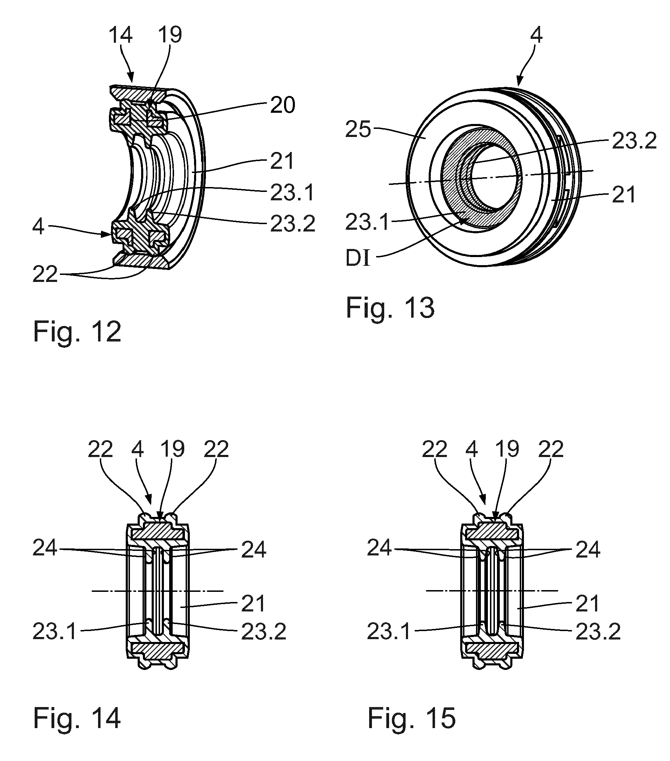

In den Fig. 13 bis 15 ist das Dichtelement 4 dargestellt, wobei das von seiner Innenöffnung nach innen abstehende Paar von Dichtlippen 23.1, 23.2 als in Fig. 13 schraffiert angedeutete Innendichtung DI eine Abdichtung zwischen zwei benachbarten Kontaktfederelementen 3 im Bereich der von der Steckerbohrung-Modulbaugruppe 1 gebildeten Steckerbohrung S sicherstellt. Diese Dichtlippen 23.1, 23.2 schließen mit einem in die Steckerbohrung S (Fig. 3, 19 und 20) eingesteckten (nicht dargestellten) Stecker beispielsweise einer Herzschrittmacherelektrode dicht ab, so dass die einzelnen Kontaktfederelemente 3 wirkungsvoll voneinander isoliert sind. Fig. 14 zeigt dabei Dichtlippen 23.1, 23.2, die eine Lippengeometrie mit parallelen Seitenwänden 24 aufweisen. Die Dichtlippen 23.1, 23.2 in der Ausführungsform gemäß Fig. 15 weisen konisch zueinanderstehende Seitenwände 24 auf.

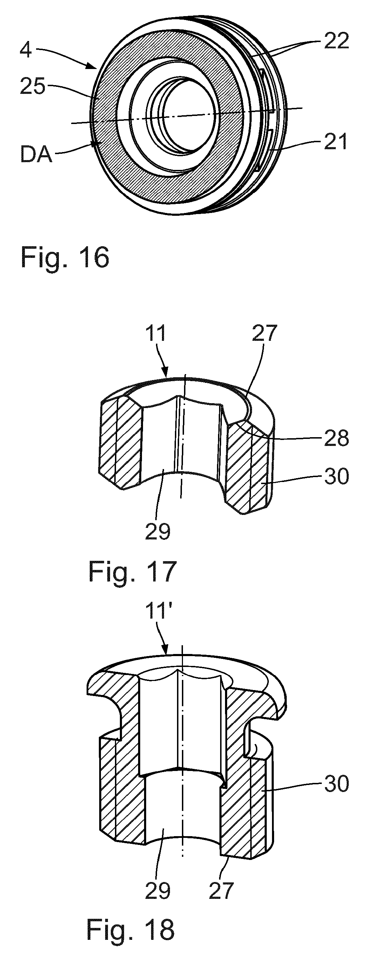

In Fig. 16 ist wiederum das Dichtelement 4 gezeigt, wobei eine in Axialrichtung A weisende, ringförmige Axialdichtung DA durch Schraffur hervorgehoben ist. Die diese Axialdichtung DA realisierende Dichtfläche 25 dichtet durch die Verspannung der Modulbaugruppe 1 mit der Spannkraft F gegen das jeweils benachbarte Kontaktfederelement 3 bzw. den Deckel 5 oder die Steckeraufnahme 2 ab.

Bei der in Fig. 14 und 15 dargestellten Version des Dichtelementes 4 tritt der Spacer 19 nicht nach außen zutage, so dass unter dem Einfluss der Spannkraft F die Dichtung 21 im Bereich der axialen Dichtfläche 25 komprimiert werden kann. Insgesamt ergibt sich für die aneinander gereihten Elemente der Steckerbohrung-Modulbaugruppe 1 also eine Art schwimmende Lagerung.

In Fig. 6 bis 9 ist das Dichtelement 4' mit teilweise freigestelltem Spacer 19 dargestellt, wobei die Dichtung 21' nur die Dichtlippen 23.1, 23.2 für eine Innendichtung DI und konvex über dem Spacer 19 in Axialrichtung A hinaus stehend die Dichtfläche 25 zur Axialabdichtung DA ausbildet. Die außerhalb der Dichtfläche 25 umlaufende Ringschulter 26 des Spacers 19 dient dabei jeweils als Anschlag beim Verpressen der Modulbaugruppe 1, so dass keine schwimmende Lagerung, sondern eine definierte Positionierung der auf mechanischen Kontakt zueinander sitzenden Komponenten innerhalb der Modulbaugruppe 1 gewährleistet ist.

In den Fig. 17 und 18 sind unterschiedliche Ausführungsformen des Gewindestiftes 11, 11' gezeigt, der in die Gewindebohrung 10 der Steckeraufnahme 2 eingesetzt ist. Bei der Herstellung der Steckerbohrung-Modulbaugruppe 1 dient der Gewindestift 11, 11' zur Fixierung des Montagedoms 7, um den verspannten Zustand der Modulbaugruppe 1 für den Verguss aufrechtzuerhalten. Insbesondere in der Ausführungsform gemäß Fig. 17 ist oben die Ringschneide 27 angedeutet, die sich durch die Fase 28 der durch die Gewindestifte 11 bzw. 11' durchgehenden Entlüftungsöffnung 29 und dem auslaufenden Gewinde 30 ergibt. Beim Einschrauben des Gewindestiftes 11 bilden sich auf dieser Ringschneide 27 Deformationen, die als Klemmmarker bezeichnet werden. Nach der Herstellung der Modulbaugruppe kann mit Hilfe dieser Klemmmarker an dem heraus geschraubten Gewindestift 11 festgestellt werden, dass die Modulbaugruppe 1 durch die entsprechende Fixierung des Montagedoms 7 mit einem hohen Drehmoment über den Gewindestift 11 so fixiert war, dass die Verspannung der einzelnen Elemente der Modulbaugruppe unter entsprechender Abdichtung aller Elemente zueinander gewährleistet war.

Im späteren Einsatz der Modulbaugruppe 1 in seiner Einbauposition in einem Schrittmacher oder anderen implantierbaren kardiologischen Gerät kann über einen entsprechenden Gewindestift 11 der in die Steckerbohrung S eingesteckte Anschlussstecker beispielsweise eines IS-1- bzw. DF-1-Systems entsprechend fixiert werden. Beim Einstecken des Steckers kann dann über die Entlüftungsöffnung 29 die in der Steckerbohrung S befindliche Luft entweichen, so dass beim Einstecken kein "Luftpumpeneffekt" mit einem entsprechenden Herausschieben des Steckers aus der Steckerbohrung S auftritt.

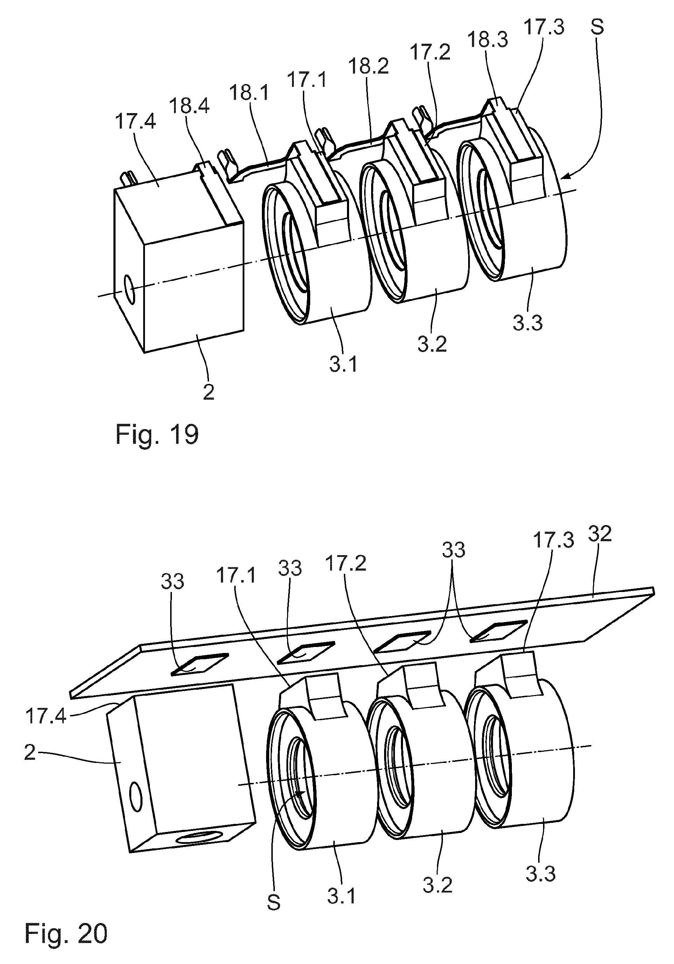

In Fig. 19 und 20 ist die Steckerbohrung-Modulbaugruppe 1 nur mit den elektrischen Anschlusselementen in Form der Kontaktfederelemente 3.1 bis 3.3 und der Steckeraufnahme 2 gezeigt. Alle weiteren Elemente sind der Übersichtlichkeit halber weggelassen. Beide Darstellungen sollen in Ergänzung zur Fig. 3 nochmals die Kontaktfederelemente 3.1 bis 3.3 mit den Plateaus 17.1 bis 17.3 zeigen, die zur elektrischen Anbindung entsprechender Kontaktbänder 18.1 bis 18.3 dienen. Die Oberseiten der Plateaus 17.1 bis 17.3 bleiben genauso wie das obere Plateau 17.4 der Steckeraufnahme 2 vom Verguss 13 frei, so dass nach Herstellung der Modulbaugruppe 1 an diesen Plateaus 17.1 bis 17.4 die Kontaktbänder 18.1 bis 18.4 angelötet oder angeschweißt werden können.

Wie in Fig. 19 nicht dargestellt ist, können mehrere Plateaus 17.1 bis 17.3 über den Umfang der Kontaktfederelemente 3.1 bis 3.3 verteilt sein, in dem beispielsweise deren Außenkontur als Vierkant ausgebildet ist.

Wie aus Fig. 20 deutlich wird, können die Plateaus 17.1 bis 17.4 auch direkt zur elektrischen und/oder mechanischen Verbindung mit einer Platine 32 dienen, wobei die Lötflächen 33 auf der Platine 32 beispielweise mit entsprechenden Signalleitungen verbunden sein können. Damit werden die Kontaktfederelemente 3.1 bis 3.3 und die Steckeraufnahme 2 nicht nur mechanisch fixiert und abgeschirmt, sondern auch signaltechnisch angebunden. Insbesondere bei der Platinenanordnungen gemäß Fig. 20 ist es von Vorteil, wenn die Plateaus 17.1 bis 17.4 in einer Ebene angeordnet sind.

## Patentansprüche

1. Verfahren zur Herstellung einer mehrpoligen, insbesondere 4-poligen Steckerbohrung-Modulbaugruppe (1) eines Herzschrittmachers oder anderen implantierbaren kardiologischen Geräts, welche Modulbaugruppe (1) eine Steckeraufnahme (2), mehrere Kontaktfedernelemente (3), dazwischen angeordnete Dichtungen (4, 4') und einen Deckel (5) umfasst, **gekennzeichnet durch** folgende Verfahrensschritte:
- axiale Aneinanderreihung der Steckeraufnahme (2), der Kontaktfederelemente (3) mit dazwischen angeordneten Dichtungselementen (4, 4') und des Deckels (5) auf einem als Fertigungshilfsmittel dienenden Montagedom (7) vorzugsweise mit einem Zwischenring (36) zur Bildung einer vorbereiteten Baugruppe,
- Einlegen der vorbereiteten Baugruppe (1) in eine Spannvorrichtung (8),
- axiales Verspannen der vorbereiteten Baugruppe (1) mit einem definierten Verspannweg oder Spannkraft (F), und
- Einlegen der vorbereiteten, verspannten Baugruppe (1) in einen Vergussmaster (12) und deren Vergießen (13) mit einem Vergussmittel zur Bildung der Steckerbohrung-Modulbaugruppe (1).

2. Verfahren nach Anspruch 1, **dadurch gekennzeichnet, dass** das axiale Verspannen der vorbereiteten Baugruppe (1) Maximalwert-begrenzt, vorzugsweise mit einem Verspannweg zwischen 0,01 mm und maximal 5 mm und/oder einer maximalen Spannkraft (F) von 200 N, ist.

3. Verfahren nach Anspruch 1 oder 2, **dadurch gekennzeichnet, dass** das Vergießen der vorbereiteten, verspannten Baugruppe (1) mit oder ohne Vakuumeinfluss in einem Druckbereich zwischen 1100 mbar und 0,1 mbar erfolgt.

4. Verfahren nach einem der vorgenannten Ansprüche, **dadurch gekennzeichnet, dass** durch das Verspannen der vorbereiteten Baugruppe (1) die Dichtungselemente (4, 4') axial verpresst werden.

5. Verfahren nach einem der vorgenannten Ansprüche, **dadurch gekennzeichnet, dass** die Dichtungselemente (4, 4') zur Realisierung einer radialen Vorspannung in eine zylindrische Bohrung einer Hülse (14.1, 14.2) oder eines Moduls einmontiert werden.

6. Verfahren nach einem der vorgenannten Ansprüche, **dadurch gekennzeichnet, dass** zum Fixieren des Montagedoms (7) beim Montageprozess ein mit einer Entlüftungsöffnung (29) versehener Gewindestift (11, 11') in die Steckeraufnahme (2) eingesetzt wird.

7. Verfahren nach Anspruch 6, **dadurch gekennzeichnet, dass** der Gewindestift (11, 11') mit einer an seinem Gewindeauslauf entstehenden Ringschneide (27) in den Montagedom (7) eingreift, wodurch eine Deformation der Ringschneide (27) zur Erhöhung der Fixierkraft und als Indiz für die Kontaktierung des Gewindestiftes erfolgt.

8. Verfahren nach einem der vorgenannten Ansprüche, **dadurch gekennzeichnet, dass** die fertig vergossene Steckerbohrung-Modulbaugruppe (1) vor ihrer Montage auf einem elektrisch aktiven Implantat, wie Herzschrittmacher oder anderen implantierbaren kardiologischen Gerät, einer funktionellen Prüfung insbesondere auf Dichtigkeit, Hochspannungsverhalten und/oder Steck- und Auszugkräfte unterzogen wird.

9. Steckerbohrung-Modulbaugruppe eines Herzschrittmachers oder anderen implantierbaren kardiologischen Geräts, hergestellt nach einem Verfahren gemäß einem der vorgenannten Ansprüche, **dadurch gekennzeichnet, dass** die Modulbaugruppe (1) eine Steckeraufnahme (2), mehrere Kontaktfedernelemente (3), dazwischen angeordnete Dichtungselemente (4) und einen Deckel (5) umfasst.

10. Steckerbohrung-Modulbaugruppe nach Anspruch 9, **dadurch gekennzeichnet, dass** die Dichtungselemente (4) aus einem Spacer (19) sowie einer darauf angeordneten Dichtung (21, 21') in Form eines Elastomerelements bestehen und vorzugsweise drei voneinander unabhängige Dichtfunktionen erfüllen, nämlich Außendichtung (DE), Innendichtung (DI) und axiale Dichtung (DA).

11. Steckerbohrung-Modulbaugruppe nach Anspruch 10, **dadurch gekennzeichnet, dass** die Dichtung (21, 21') aus einem Material, vorzugsweise einem biokompatiblen Zwei-Komponenten-Gemisch, besteht, dessen Shorehärte zwischen 20 und 70 Shore A liegt und/oder **dass** der Spacer (19) aus einem Epoxydharz, PEEK, Polysulfon, Polyurethan, Polyurethan Copolymer, Silikon, Keramik, Polyimide und/oder deren Mischformen besteht.

12. Steckerbohrung-Modulbaugruppe nach einem der Ansprüche 9 bis 11, **dadurch gekennzeichnet, dass** die Dichtung (21, 21') an den Spacer (19) angespritzt ist, wobei vorzugsweise
- Durchbrüche (20) im Spacer (19) vorgesehen sind, durch die das Elastomer der Dichtung (21, 21') gespritzt ist,
- der Spacer (19) zur Verbesserung des Haftvermögens durch Prozesse, wie Definition der Oberflächenrauheit, Plasmaaktivieren, nasschemisches Benetzen oder CO₂-Strahlen vorbereitet ist, und/oder
zwischen der Dichtung (21, 21') vorzugsweise aus Silikon und dem Spacer (19) vorzugsweise aus PEEK Nanokristalle vorzugsweise in Form von Zinkoxidkristallen mit einer Größe von mehreren Nano- bis wenigen Mikrometern zur Haftverbesserung angeordnet sind..

13. Steckerbohrung-Modulbaugruppe nach einem der Ansprüche 9 bis 12, **dadurch gekennzeichnet, dass** das Dichtungselement (4) in eine Hülse (14.1, 14.2) eingesetzt ist und als Außendichtung mit Funktionsflächen (22) mit der Hülse (14.1, 14.2) abdichtet.

14. Steckerbohrung-Modulbaugruppe nach einem der Ansprüche 9 bis 13, **dadurch gekennzeichnet, dass** das Dichtungselement (4) eine Innendichtung vorzugsweise in Form eines parallelen oder konischen Dichtlippenpaares (23.1, 23.2) aufweist.

15. Steckerbohrung-Modulbaugruppe nach Anspruch 14, **dadurch gekennzeichnet, dass** die Innendichtung (23.1, 23.2) mit einer Beschichtung zur Verringerung der Steckkräfte versehen ist.

16. Steckerbohrung-Modulbaugruppe nach einem der Ansprüche 9 bis 15, **dadurch gekennzeichnet, dass** das Dichtungselement (4) eine Axialdichtung (25) aufweist, die beidseitig durch eine konvexe Auslegung nach erfolgter axialer Verspannung mit den weiteren Komponenten (2, 3, 5) der Modulbaugruppe (1) abdichtet.

17. Steckerbohrung-Modulbaugruppe nach Anspruch 16, **dadurch gekennzeichnet, dass** ein fester Anschlag (26) am Spacer (19) vorgesehen ist, mit dem die Positionsabstände der Komponenten eindeutig einstellbar sind.

18. Steckerbohrung-Modulbaugruppe nach einem der Ansprüche 9 bis 17, **dadurch gekennzeichnet, dass** die Modulbaugruppe (1) in der Steckeraufnahme (2) einen Gewindestift (11, 11') aufweist, der mit einer Entlüftungsöffnung (29) versehen ist und der während des Montageprozesses für die Steckerbohrung-Modulbaugruppe (1) zur Fixierung gegenüber dem Montagedom (7) sowie im fertiggestellten Produkt selbst zur Fixierung gegenüber einem in die Steckerbohrung (S) eingesetzten Stecker mit gleicher Funktionalität zum Einsatz kommt.

19. Steckerbohrung-Modulbaugruppe nach einem der Ansprüche 9 bis 18, **dadurch gekennzeichnet, dass** die Kontaktfedernelemente (3) mit mindestens einem Schweiß- oder Lötplateau (17) zur Anbindung von Anschlusselementen, vorzugsweise Anschlussbändern (18) oder einer lötfähigen Platine (32), oder von Fixierungen oder von Abschirmungen versehen sind.

20. Steckerbohrung-Modulbaugruppe nach Anspruch 19, **dadurch gekennzeichnet, dass** das Schweiß- oder Lötplateau (17) an den Kontaktfederelementen (3) sich auf einem Höhenniveau mit einem Schweiß- oder Lötplateau (17.4) der Steckeraufnahme (2) befindet.

21. Steckerbohrung-Modulbaugruppe nach einem der Ansprüche 9 bis 20, **dadurch gekennzeichnet, dass** das Vergussmittel des Vergusses (13) aus einem Harz, einem Polyurethan, einem Silikon oder deren Gemischen besteht.
